# EUROPEAN PATENT SPECIFICATION

(11) **EP 0 493 380 B1**
(45) Date of publication and mention of the grant of the patent: **29.10.1997**
(21) Application number: 90902584.3
(22) Date of filing: 05.09.1989
(51) Int. Cl.: A61K 9/00

(54) **METHOD AND COMPOSITIONS FOR NONINVASIVE DOSE-TO-EFFECT ADMINISTRATION OF LIPOPHILIC DRUGS**
VERFAHREN UND ZUSAMMENSETZUNGEN ZUR VERABREICHUNG VON LIPOPHILEN HEILMITTELN IN EINER DOSIERUNG NACH DER GEWÜNSCHTEN WIRKUNG
METHODE DE PREPARATION PERMETTANT L'ADMINISTRATION NON INVASIVE DE MEDICAMENTS LIPOPHILES DOSES EN FONCTION DE L'EFFET RECHERCHE

(43) Date of publication of application: 08.07.1992
(73) Proprietor: UNIVERSITY OF UTAH RESEARCH INSTITUTE, Salt Lake City, Utah 84112 (US)
(72) Inventor: STANLEY, Theodore, H., Salt Lake City, UT 84124 (US); HAGUE, Brian, West Valley City, UT 84119 (US)
(74) Representative: Browne, Robin Forsythe, Dr.
(86) International application number: US8903801
(87) International publication number: WO9103233

(56) References cited:
- EP-A- 0 107 941
- EP-A- 0 200 490
- US-A- 2 208 120
- US-A- 3 943 928
- US-A- 4 551 329
- US-A- 4 671 953
- ANESTHESIA AND ANALGESIA, vol. 69, no. 1, July 1989, pages 21-27; T.H. STANLEY et al.: "Oral transmucosal fentanyl citrate (lollipop) premedication in human volunteers"

## Description

### BACKGROUND

The present invention is related to compositions for use in delivering pharmacological agents to a patient. The present invention relates particularly but not exclusively to compositions for the noninvasive administration of precisely the proper dose of potent pharmacological agents having antimigraine, antiemetic or bronchodilator properties.

In recent years, a host of potent new drugs have become available for clinical use in treating migraine headaches, nausea, vomiting, asthma, respiratory distress or polyuria. Current expectations are that additional potent drugs will continue to become available in the future.

In addition to treating specific diseases and conditions, physicians can prescribe drugs that will permit the physician to regulate many body functions and processes. Yet, despite the tremendous advances in the field of pharmacology, physicians continue to administer these new drugs using substantially the same techniques that have been employed for many decades.

Thus, almost all pharmacological agents continue to be administered via two routes, by oral administration for absorption through the stomach or intestines or by intramuscular or intravenous injection, despite the fact that both of these routes suffer from significant disadvantages under typical situations.

The simplest and most prevalent administration route is oral administration. To use this method, a pharmacological agent is incorporated into a tablet, a capsule, or into a liquid base; the patient then ingests an appropriate dose. Oral administration of a drug is extremely convenient, and for many drugs, it will continue to be the method of choice. Such administration is nonthreatening and is painless to the patient. For most patients, it is also very simple.

Nevertheless, oral administration of a drug suffers from the disadvantage that pediatric and geriatric patients frequently have difficulty swallowing pills, and such patients often refuse to cooperate in swallowing a liquid medication. Even more importantly, absorption of a drug into the bloodstream after swallowing a tablet varies from patient to patient and in the same patient from time to time. The absorption of the drug is dependent upon the movement from the stomach to the small and large intestines and the effects of secretions from these organs.

Moreover, there is often a substantial delay between the time of oral administration of a drug until it begins to have the desired therapeutic effect on the patient's system. Generally, a drug must pass from the stomach into the small and large intestines before it will be absorbed into the patient's bloodstream; unfortunately, this typically takes forty-five minutes or longer. For some applications, such a delay is unacceptable.

Further, many drugs taken orally are metabolized almost immediately -- they are removed from or rendered ineffective by the patient's system before they can have any therapeutic effect. This occurs because the veins from the stomach and the small and large intestines drain into the liver. Thus, drugs entering the patient's bloodstream through the stomach and the intestines immediately pass through the patient's liver before distribution throughout the remainder of the patient's body.

Unfortunately, upwards of sixty percent of a drug (and essentially one hundred percent of certain drugs) may be removed from the patient's bloodstream during this first pass through the liver. Therefore, when utilizing oral administration, much larger doses of the drug than would otherwise be necessary must be administered to the patient to compensate for the large percentage of the drug removed during the first pass through the liver and obtain the desired effect in the patient.

Adverse reactions from the large doses necessary to elicit a systemic or local effect despite metabolism by the liver include nausea, vomiting, involuntary movement, gastrointestinal bleeding, duodenal ulcers, and epigastric abdominal distress. Other dose-related side effects may adversely affect renal and hepatic functions, especially in the geriatric patient. Some drugs, if present in the liver in excess, may also be hepatoxic.

The result is that the oral route of administration is inefficient for many drugs, particularly many antimigraine, antiemetic, brochodilator,

In some instances, a dose approximately one hundred times the actual effective dose must be administered to the patient in order to retain a sufficient dose of the drug in the blood after the first pass through the liver. Thus, oral adminstration results in a highly inefficient use of the drug. Further, in addition to the other adverse effects resulting from large amounts of the drug being removed by the liver, oral administration is also disadvantageous because of the cost in providing such a large dose of the drug.

A further difficulty encountered when administering drugs orally is that dosages are prepared or determined for use with an "average" patient. This is entirely acceptable for many drugs, but some drugs have a widely varying effect on different patients, even when weight and size differences between patients are considered. The effects of these drugs can vary depending upon the patient's habits, subtle genetic differences between patients, the patient's blood volume, the patient's age, and numerous other known and unknown individual variations in susceptibility to the particular drug utilized.

Underdosing a patient because of a low susceptibility to the drug fails to evoke the response sought by the physician. Overdosing the patient can result in dangerous depression of vital body functions. Moreover, the slow and uncertain response time for the onset of an observable reaction to a drug when taken orally makes it even more difficult to determine a proper dose for a particular patient. The physician may not learn for an hour, or with some drugs for a few days, whether the patient was underdosed or overdosed.

In order to avoid these serious disadvantages inherent in the oral administration modality, physicians frequently resort to the injection modality for administering many drugs. Injecting a drug (generally intravenously or intramuscularly) results in rapid entry of the drug into the patient's bloodstream; in addition, this type of delivery avoids the removal of large quantities of the drug by the patient's liver that accompanies oral administration. Rather, the drug becomes rapidly distributed to various portions of the patient's body before exposure to the liver; thus, the drug is removed by the liver at a substantially slower rate.

Most patients have at least some aversion to receiving injections. In some patients, particularly children and certain "high strung" adults, this aversion may be so pronounced as to make the use of injections of serious concern to the physician. Since intense psychological stress and anxiolysis can exacerbate a patient's debilitated condition, it sometimes becomes undesirable to use injections where the patient is seriously ill or suffers from a debilitating condition or injury.

To compound the problem facing a physician, the individual variation in susceptibility and metabolism with respect to various drugs, which makes it difficult to select an appropriate dose for oral administration, is even more profound when utilizing the injection modality of administration. This is because smaller doses have an increased effect due to the rapidity with which the drug enters the bloodstream and because large doses of the drug, when injected, are not immediately metabolized by the liver.

In order to prevent overdosing a patient with potent drugs, a prudent physician typically injects a patient with a lower than average dose, and later supplements the dose with additional injections as they appear necessary. This "titration" makes necessary the use of repeated injections, which in turn greatly increase the stress on the patient. It is not uncommon for a patient to come to fear that it is time for yet another injection every time the patient sees a member of the hospital staff, which is often the case for those most in need of potent drugs.

In view of the foregoing, it will be appreciated that it would be an important advancement in the art of administering drugs if suitable methods and compositions could be provided for administering drugs in order to provide for rapid onset of the desired action and precise dosage delivery in each patient so as to avoid the dangers of overdosage and underdosage.

It will also be appreciated that it would be an important advancement in the art of administering drugs if suitable methods and compositions could be provided that avoid immediate metabolism of the drug trough the patient's liver and yet not involve injection of the drug.

It would be an additional important advancement if methods and compositions could be provided that would permit a physician and/or a patient to easily control the amount of the drug the patient receives according to the patient's own subjective need for medication.

Such methods and compositions are disclosed and claimed herein.

According to the present invention there is provided a composition for use in treating in a dose-to-effect manner a patient experiencing pain associated with a migraine, said composition comprising:
an effective dose of a drug capable of being absorbed through mucosal tissues of the mouth, pharynx, and oesophagus, and capable of rapidly relieving the pain associated with a migraine;
a soluble matrix material, the drug being dispersed substantially uniformly within the matrix so that the drug is released in a dose-to-effect manner for absorption through mucosal tissues of the mouth, pharynx, and oesophagus as the matrix dissolves when placed in a patient's mouth; and
holder means secured to the drug-containing matrix, said holder means being configured so as to permit convenient insertion of the drug-containing matrix into the mouth of a patient and convenient removal thereof while accounting for the patient's susceptibility to the drug and the patient's individual subjective experience of the pain associated with the migraine.

Even patients that have difficulty swallowing a pill or tablet or refuse to swallow a pill, tablet, and/or a liquid, will give little resistance to sucking on a lollipop. Particularly when dealing with children, a lollipop evokes a pleasurable response in the patient and gives the patient something non-threatening on which to concentrate.

An antimigraine, antiemetic, bronchodilator, acting drug administered by way of a lollipop will quickly enter the patient's bloodstream through the veins which serve the mucosal tissues in the mouth and pharynx, thereby serving to further lessen any remaining tension and fear. Appropriate monitoring of the patient's reaction (eg pain, asthma, urine output, etc) to these potent drugs will indicate when the drug has evoked a suitable response. The lollipop may then be removed, or its rate of consumption may be decreased.

It will be appreciated that the ever-present risk of overdosing a patient is substantially minimized, if not almost eliminated, by the dose-to-effect administration method of the present invention. The rate at which the drug is to be absorbed by the body can be varied by varying the rate the lollipop dissolves.

Accordingly, the drug dose is given over a period of time rather than all at once (as in a pill or other bolus injection), and the administration rate can be reduced if such appears necessary. If a patient begins showing signs of any overdose, he will simply stop sucking the lollipop and/or the physician can easily remove the lollipop from the patient's mouth.

Unlike the use of injections or oral ingestion of medication where a relatively large bolus dose of medication is given intermittently, use of a lollipop can permit the patient to take very small doses of a drug on an almost continuous basis. Moreover, such administration can be regulated in response to the patient's own need for medication in light of his own subjective experience and his own personal susceptibility to the particular drug utilized. The result is that lower amounts of drugs can be used to achieve a more even medication of the patient.

It is, therefore, a preferred aspect of the present invention to provide noninvasive compositions capable of rapidly inducing an antimigraine, antiemetic or bronchodilator effect through the dose-to-effect adminstration of appropriate drugs without the dangers of overdosage or underdosage.

It is another preferred aspect of the present invention to provide compositions that would allow for more physician control over the administration of these potent drugs so that individual patient differences in susceptibility and metabolism can be taken into account.

Yet another aspect of the present invention is to provide compositions for drug administration which minimize the psychological trauma generally associated with injections and the adverse physical and psychological problems often associated with the oral administration of potent drugs.

Yet another aspect of the present invention is to provide compositions that will permit a patient to control the amount of medication administered according to the individual variations in the susceptibility to the particular medication used and in response to the patient's subjective experience of pain or other biological indicators.

These and other features of the present invention will become fully apparent from the following description and appended claims.

While maintaining the convenience or oral administration, the present invention provides for many of the advantages of the injection modality of drug administration. At the same time, the present invention avoids the disadvantages identified above with respect to these two traditional routes of adminstration. The present invention achieves these results by utilizing yet a third adminstration route -- absorption through the mucosal tissues in the mouth and around the pharynx and oesophagus.

A very few drugs, such as nitroglycerin, have historically been administered by absorption through mucous tissue because the transmucosal route is faster than oral administration, and unlike injections can be easily self-administered. While such drugs are easily given by the transmucosal route, they have not, unfortunately, been given by a dose-to-effect method. In dose-to-effect drug administration, the drug is administered until a predetermined effect is obtained; thereafter, the administration process is modified or terminated. By contrast, the prior art has consistently utilized the procedure of administering a bolus of the drug. In ANESTHESIA AND ANALGESIA, vol.69, n.1, July 1989, pages 4-22, and EP-A-0 200 490 the authors of the present invention have incorporated drugs having a sedative, analgesic or anaesthetic effect in the form of a lollipop. Also US-A-2 208 120 discloses preparations in the form of a pop containing acetyl salicylic acid and bromatological salts.

Despite some limited use, the transmucosal route has not been favored for routine use. Instead, where a delay in drug action is acceptable, the oral route has been preferred by most physicians, and injections have been used where delay is not acceptable.

Transmucosal dose-to-effect delivery of a drug is somewhat slower to provide active concentrations of a drug in a patient's system than is the use of an intravenous injection. Nevertheless, it has been discovered that the transmucosal route can be adapted so that any loss in the speed of drug uptake is more than offset by the ability to administer the drug noninvasively (much to the appreciation of the patient) and by the ability to control the dose received by the patient vis-a-vis the effect of the drug.

A drug must be lipophilic in order to be absorbed across mucosal tissue. However, this requirement is not a serious limitation since a large number of drugs are naturally lipophilic or can be provided in a lipophilic form.

In accordance with the present invention, a suitable drug is dispersed within a carbohydrate mass, a compressed powder form, or other suitable matrix in the form of a lollipop. (The method of manufacturing a suitable compressed powder lollipop which is usable for certain potent drugs within the scope of the present invention is described in detail in U.S. patent application Serial No. 07/060,045, filed Jun 8, 1987, in the name of the inventors hereof, and entitled "COMPOSITIONS AND METHODS OF MANUFACTURE OF COMPRESSED POWDER MEDICAMENTS."

The drug-containing lollipop is then given to a patient to suck on so that the drug will be released into the patient's mouth as the carbohydrate mass or compressed powder matrix dissolves. Being lipophilic, a significant portion of the drug is absorbed into and/or through the mucosal tissues of the mouth, pharyngeal, and esophageal areas. The drug rapidly enters the patient's bloodstream, and importantly, the blood in the veins draining from the mouth and the pharyngeal and esophageal areas passes through the central nervous system and then through a substantial portion of the body (so that the drug can be absorbed) before the blood passes through the liver (where most of the drug is inactivated). In case of a localize effect, the drug quickly contacts the affected area in order to provide effective relief as soon as possible, while minimizing the amount of the drug administered to the patient.

The use of a carbohydrate or compressed powder matrix (i.e., "candy") to administer a drug offers some important advantages, particularly when dealing with paediatric patients. First, a candy lollipop is familiar and lacks the menace of a syringe and needle. Being a substance normally associated with pleasure, the drug-containing candy lollipop immediately evokes a positive psychological response.

Importantly, it has been found that the use of drug-containing candy in the form of a lollipop can permit the physician to control the dosage of the drug administered to the patient in order to relieve a migraine headache, asthma, nausea, vomiting, or bronchospasm, or to induce childbirth, increase urine output, relieve the effects of Parkinson's disease, or treat systemic or oral fungal infections, polyuria or gastric and duodenal ulcers, thereby resulting in dose-to-effect drug administration. Use of such drug-containing lollipops also permits the patient in certain circumstances to exert control over the dosage received of certain medication in order to diminish feelings of discomfort or pain.

These important advantages are available because very small amounts of a potent drug may be delivered to a patient substantially continuously, and administration of the drug may be halted at any time by simply removing the candy from the patient's mouth. This not only allows a physician to monitor a patient's condition so that a particular effect is obtained and maintained, but also provides the important safety benefit of reducing the risk of overdose.

It is much less likely that a patient receiving medication in accordance with the dose-to-effect method of the present invention will become overdosed since the dose builds relatively slowly until a desired effect is achieved. Further, if a patient becomes slightly overdosed, it is likely that the patient will stop sucking the drug-containing lollipop and/or the physician or other medical personnel will observe the situation and remove the lollipop before the patient becomes seriously overdosed.

In contrast, once a typically large dose of a drug is given orally, by injection, or even sublingually or nasally, there is no retrieving it; thus, the full effects of the administered drug will be felt. Further, a large dose given every few hours results in wide swings in plasma concentration of the drug, while the use of a lollipop in accordance with the present invention evens out the plasma concentration of that drug.

In practice, a physician can offer the patient a piece of medicated candy on a holder, together with simple instructions that the candy is to be sucked rather than chewed. Children will particularly be put at ease by this approach but so will anxious adults. The physician can then monitor the patient's condition to ensure the desired effect is achieved.

As mentioned above, it is preferred that the medicated candy take the form of a lollipop. Use of a stick or other suitable holder permits easy removal of the candy when a physician deems that a patient has received a proper dosage of the drug contained therein. Provision of a suitable holder also facilitates intermittent administration of a drug to maintain a desired condition and makes it more convenient for a patient to intermittently self-administer the drug in response to variations in the patient's subjective perception of physiologic condition.

The speed at which a sufficient amount of drug enters the patient's bloodstream so as to produce a desired effect depends on several factors. For example, a very potent drug requires fewer drug molecules to enter the patient's system than does a weak drug to produce a desired effect. Accordingly, if rapid onset of bronchodilation is desired, a potent rather than a weak drug could be used.

Additionally, the degree of lipophilicity of a drug directly affects the rate of absorption of the drug. A highly lipophilic drug will result in the more rapid onset of a desired patient response than will a more moderately lipophilic drug. For example, ergotrate is a very potent drug which is highly lipophilic. However, ergonovine is nearly twice as lipophilic as ergotrate and thus is capable of faster absorption. It will be appreciated, however, that other pharmacokinetic properties of a drug will affect the rate at which the effect of the drug is observed in the patient.

The choices of matrix and the concentration of the drug in the matrix are also important factors with respect to the rate of drug uptake. A matrix that dissolves quickly will deliver drug into the patient's mouth for absorption more quickly than a matrix that is slow to dissolve. Similarly, a candy that contains a drug in a high concentration will release more drug in a given period of time than a candy having a low drug concentration.

It will be appreciated that varying the concentrations of the drug in the matrix or the properties of the matrix (particularly the rate at which the matrix dissolves) can be advantageously used in designing specific compositions for specific uses. A lollipop containing meclizine of a given concentration may be used to relieve nausea, while a lollipop having a stronger concentration (and preferably a different color so as to prevent confusion) may be used when it is desired to relieve vomiting or emesis.

Another use of these properties is to prepare a multi-layer lollipop where the outer layer is of a concentration differing from that of the inner layer. Such a drug delivery system has a variety of applications. By way of example, it may be desirable to quickly get a predetermined dose of a drug into the bloodstream to obtain a desired effect and then use a different concentration to maintain that effect.

The choice of a particular carbohydrate or compressed powder matrix is subject to wide variation. Conventional sweeteners such as sucrose or corn syrup may be utilized, or carbohydrate suitable for use with diabetic patients, such as sorbitol or mannitol might be employed. Other sweeteners, such as aspartame, can also be easily incorporated into a composition in accordance with the present invention. The candy base may be very soft and fast-dissolving, or may be hard and slower-dissolving. Various forms will have advantages in different situations.

It will be appreciated that all suitable drugs within the scope of the present invention may be prepared in the compressed powder form whereas only those drugs with relatively high melting points may be prepared in the more traditional hard candy form. It has been found that both forms operate in substantially the same way. Typical examples illustrating the method of preparing a hard candy matrix and a compressed powder candy matrix are given herein below. Example 1, not within the present invention, illustrates manufacture of the claimed dosage forms.

### EXAMPLE 1

The candy matrix or base for the drug-containing lollipop within the scope of the present invention is advantageously prepared utilizing candy preparation formulas and techniques which are known in the prior art. For example, a hard candy base is prepared by dissolving 50 grams of sucrose in 50 grams of water and heating the solution to about 115°C (240°F). Next, about 40 grams of corn syrup having a dextrose equivalent of 42 units, and a high maltose content (30%-35% maltose) is added, and the mixture is cooked at about 140°C (300°F) to reduce the water content to about three percent (3%). After recooling the thickened candy mass to about 115°C (240°F), a suitable oil flavouring (eg lemon or cherry) is added.

Concurrently, a solution containing a soluble drug is prepared for incorporation into a candy matrix. In this example, the drug selected is clotrimazole. Clotrimazole is a potent antifungal agent useful in treating oral candidiasis or monoliasis. Its high potency and lipophilicity make it an excellent drug for transmucosal administration in accordance with the present invention.

A suitable clotrimazole solution is prepared by dissolving 200 milligrams of clotrimazole in 10 cubic centimetres of sterile ethanol. This clotrimazole solution is mixed with 32 cubic centimetres of the hot candy mass formed as set forth above, and the resultant mixture is gently mixed as it cools to about 117°C (225°F), taking care not to induce formation of air bubbles in the candy mass.

The solution is then poured into suitable molds having a 2.0 cubic centimetre capacity that have been prelubricated with vegetable oil to prevent sticking. A four inch commercially available wax-coated compressed paper stick is next inserted into the base of each mold. The mixture is then permitted to set.

The foregoing procedure results in the preparation of 20 lollipops, each containing 10 milligrams of clotrimazole.

### EXAMPLE 2

In this example, ergotamine is selected for incorporation into a compressed dosage form. Ergotamine is a potent lipophilic drug useful for relieving the pain associated with migraines. Its high potency and lipophilicity make it an excellent drug for transmucosal administration in accordance with the present invention.

A suitable matrix is prepared by combining 40 milligrams of ergotamine; 5.22 grams compressible sugar; 10.44 grams maltodextran; 300 milligrams ribotide, 400 milligrams aspartame, 800 milligrams compritol 888, 1.0 grams artificial vanilla cream, 200 milligrams natural mint; 600 milligrams cherry, and 1.0 grams artificial vanilla. Alloquats of 2000 milligrams each are then hydraulically compressed around a commercially available wax-coated compressed paper holder, using a force sufficient to provide a final volume of 2 cubic centimeters. The foregoing procedure results in the preparation of 10 lollipops, each containing 4 milligrams of ergotamine.

In addition to modifying the physical characteristics of the lollipop, the technique used by the patient to suck the lollipop may also be used to affect the rate of the absorption of the drug. If substantial portions of dissolved candy and drug are swallowed, the normal complications of oral administration will be encountered (i.e., slow response and loss of drug in the stomach and liver).

If the candy is sucked slowly with little production of saliva, very little drug will be swallowed, but a reduction in the amount of saliva will also cause a reduction in the rate at which the medicated candy dissolves. It will be appreciated that the technique of sucking utilized can have a significant effect on the rate of drug uptake into the patient's bloodstream.

Use of a lollipop, in contrast to a simple drop or pellet, helps control proper placement of the candy within the patient's mouth since the physician, nurse, or even the patient can manipulate the candy. Accordingly, the medical professional can easily monitor placement by observation of the angle of the protruding stick. Once a suitable technique for sucking the lollipop has been selected, the remaining factors can be adjusted accordingly.

It will be appreciated from the foregoing that the present invention has broad applicability to a variety of antimigraine, antiemetic or bronchodilator agents. For example, the present invention may be utilized in the administration of antimigraine agents such as ergotamine, methysergide, propranolol, or suloctidil; bronchodilators such as albuterol, animophylline, beclomethasone, dyphylline, epinephrine, flunisolide, isoetharine, isoproterenol HCl, metaproterenol, oxitriphylline, terbutaline, and theophylline; antiemetic agents such as benzquinamide, meclizine, metoclopramide, prochlorperazine, and trimethobenzamide.

It will be appreciated that other drugs may also be utilized within the scope of the present invention. What is important is that the drug be lipophilic, potent, and fast-acting so that the desired effects can be observed by the medical professional (or the patient himself if the drug is self-administered) in sufficient time to remove the lollipop from the patient's mouth in time to prevent overdosing.

In incorporating a drug into a lollipop within the scope of the present invention, the amount of the drug used will generally differ from the amount used in more traditional injection and oral administration techniques. Depending upon the lipophilic nature of the drug, its water solubility, its potency, and its end use, the total concentration of the drug in a typical lollipop may contain from one to fifty times the amount of the drug which may be used in an injection.

However, for purposes of example, Table I sets forth presently contemplated ranges of the dosages of certain drugs which would typically be used.

**Table I**

| Antiemetic | |
|---|---|
| Drug Generic | Lollipop Dose Range / mg |
| Benzquinamide | 25-100 |
| Meclizine | 25-100 |
| Metoclopramide | 5-20 |
| Prochlorperazine | 5-25 |

| Bronchodilator | |
|---|---|
| Drug Generic | Lollipop Dose Range / mg |
| Albuterol | 0.8-1.6 |
| Aminophylline | 100-500 |
| Beclomethasone | 20-50 |
| Dyphylline | 100-400 |
| Epinephrine | 200-500 |
| Flunisolide | 25-50 |
| Isoetharine | 170-680 |
| Isoproterenol HCl | 60-260 |
| Metaproterenol | 0.65-10 |
| Oxitriphylline | 50-400 |
| Terbutaline | 2.5-10 |
| Theophylline | 50-400 |

| Antimigraine | |
|---|---|
| Drug Generic | Lollipop Dose Range / mg |
| Ergotamine | 2-4 |
| Methysergide | 2-4 |
| Propranolol | 80-160 |
| Suloctidil | 200-300 |

It will be appreciated from the foregoing that the present invention has broad applicability and will be useful in a wide variety of situations. It provides a useful alternative to the traditional oral and injection routes of administration, and permits the physician extraordinary control over the dosage of a potent antimigraine, antiemetic, or bronchodilator drug that is administered to a patient.

Some of the more important features and advantages of the present invention as applied to the above drug classes will be better appreciated and understood by reference to the specific discussion below:

### A. Bronchodilator Agents

Respiratory smooth muscle relaxants are used to produce relief of bronchospasm and increase respiratory flow rates and vital capacity. Theophylline is a typical bronchodilator commonly used for the symptomatic treatment of asthma and reversible bronchospasm that may occur in association with chronic bronchitis or emphysema.

Following oral administration of theophylline capsules or uncoated tablets, peak serum concentrations are usually reached in one to two hours. Peak serum theophylline concentrations are usually obtained after about one hour when theophylline oral solutions or microcrystalline tablets are administered. Absorption of theophyllines may be delayed, but generally not reduced, by the presence of food in the gastrointestinal tract. When administered intramuscularly, theophylline is usually absorbed slowly and incompletely. In addition, rectal suppositories are slowly and erratically absorbed.

In maintenance-dose theophylline schedules, serum concentrations among patients vary at least six-fold and serum half-lives exhibit wide interpatient variation because of differences in the rate of metabolism. (The elimination half-life is the time required for the plasma drug concentration to decrease by one-half.) Serum half-life ranges from about 3 to 12.8 (average 7-9 hours) in otherwise healthy, nonsmoking asthmatic adults, from about 1.5 to 9.5 hours in children, and from about 15 to 58 hours in premature infants.

When compared with that of otherwise healthy nonsmoking asthmatic adults, the serum half-life of theophylline may be increased and total body clearance decreased in patients with congestive heart failure, chronic obstructive pulmonary disease, cor pulmonale, or liver disease, and in geriatric patients. In cigarette and/or marijuana smokers, theophylline serum half-life averages 4-5 hours and total body clearance is increased compared with nonsmokers.

Therefore, due to the wide variation in patient metabolism of theophylline to administer to proper dose required by each patient. Too much theophylline may result in nausea, vomiting, headache, nervousness, seizures, sinus tachycardia, hypotension, circulatory failure or ventricular arrhythmias. If insufficient theophylline is administered, the respiratory distress continues, which can be as serious as receiving an overdose.

Hence, a theophylline-containing lollipop administered in a dose-to-effect manner would be capable of providing rapid relief of asthma or bronchospasm associated with respiratory distress. The precise dosage necessary to achieve a precise effect in each individual patient could be provided thereby substantially reducing the risk of severe consequences associated with an underdose or overdose. Thus, the wide variation in the rate of metabolism from patient to patient would be accounted for by the dose-to-effect modality.

Examples of compositions and methods of using lollipops containing bronchodilator agents are given herein below.

### EXAMPLE 3

A drug-containing lollipop within the scope of the present invention to be used in the treatment of respiratory distress is made according to the procedure of Example 2, except that the ingredients are combined in the following amounts:

| Ingredient | % | grams |
|---|---|---|
| Natural mint | 1.0% | 0.2 |
| Ribotide | 1.5% | 0.3 |
| Aspartame | 2.0% | 0.4 |
| Wild cherry | 3.0% | 0.6 |
| Compritol 888 | 4.0% | 0.8 |
| Artificial vanilla | 5.0% | 1.0 |
| Artificial vanilla cream | 5.0% | 1.0 |
| Oxtriphylline | 10.0% | 2.0 |
| Compressed sugar | 22.83% | 4.57 |
| Maltodextrin | 45.67% | 9.13 |

The foregoing procedure results in the preparation of 10 lollipops, each containing 200 milligrams of oxtriphylline.

### EXAMPLE 4

In the procedure of this example, a patient who is presently experiencing the respiratory distress is given an oxtriphylline-containing lollipop in order to rapidly relieve the respiratory distress. In this example, oxtriphylline in a lollipop dose of 200 milligrams is used. As the patient sucks on the lollipop, the symptoms of respiratory distress are rapidly relieved and the patient returns to more normal respiratory function.

### EXAMPLE 5

A drug-containing lollipop within the scope of the present invention to be used in the treatment of respiratory distress is made according to the procedure of Example 1, except that an oxtriphylline solution, in which 4 grams of oxtriphylline is dissolved in 10 cubic centimeters of water, is substituted for the clotrimazole solution. This procedure results in the preparation of 20 lollipops, each containing 200 milligrams of oxtriphylline.

Although the above discussion focused on the bronchodilators theophylline and oxtryphilline, it will be appreciated that other bronchodilators may also be utilized within the scope of the present invention. What is important is that the bronchodilator be lipophilic, potent, and fast-acting so that the desired effects can be observed by the medical professional or by the patient himself, if the drug is to be self-administered, in sufficient time to remove the lollipop from the patient's mouth in time to prevent overdosing.

### D. Antimigraine Agents

Migraines refer to sudden periodic attacks of throbbing headaches which begin in childhood, adolescence, or early adult life and continue to reoccur with diminishing frequency during advancing years. A migraine may last from about five minutes to fifteen minutes, followed by hemicranial headache, nausea, and vomiting, all of which last for hours or as long as a day or two.

The drug ergotamine provides symptomatic relief from the pain of a migraine. Ergotamine is usually administered orally or sublingually, 2 milligrams taken as soon as the headache starts, with subsequent doses of 2 milligrams at intervals of 30 minutes thereafter, if necessary, until a total of 6 milligrams has been taken. No more than 10 milligrams should be ingested per week. Since overdosage is the chief cause of untoward effects from ergotamine, the smallest amount effective for relief of the headache should be employed.

The speed and thoroughness of the relief from pain are directly proportional to the promptness with which medication is started after the onset of an attack. If the drug is given early, the dose may be decreased considerably. But if the headache has reached its peak, large quantities of ergotamine are needed. Not only is a longer time than required for effective action needed, but also undesirable side effects from this medication are more pronounced.

An ergotamine-containing lollipop administered in a dose-to-effect manner provides the precise dosage necessary to obtain rapid symptomatic relief from migraine pain while at the same time minimizing potential side effects.

Examples of compositions and methods of using lollipops containing antimigraine agents are given herein below.

### EXAMPLE 6

A drug-containing lollipop within the scope of the present invention to be used in the treatment of a migraine is made according to the procedure of Example 2, except that the ingredients are combined in the following amounts:

| Ingredient | % | grams |
|---|---|---|
| Natural mint | 1.0% | 0.2 |
| Ribotide | 1.5% | 0.3 |
| Aspartame | 2.0% | 0.4 |
| Wild cherry | 3.0% | 0.6 |
| Propranolol | 4.0% | 0.8 |
| Compritol 888 | 4.0% | 0.8 |
| Artificial vanilla | 5.0% | 1.0 |
| Artificial vanilla cream | 5.0% | 1.0 |
| Compressed sugar | 25.33% | 5.07 |
| Maltodextrin | 50.67% | 10.13 |

The foregoing procedure results in the preparation of 10 lollipops, each containing 80 milligrams of propranolol.

### EXAMPLE 7

In the procedure of this example, a patient who is presently experiencing the pain associated with a migraine is given an ergatomine-containing lollipop in order to rapidly relieve the pain associated with the migraine. In this example, ergotamine in a lollipop dose of 4 milligrams is used. As the patient sucks on the lollipop, the pain associated with the migraine is rapidly relieved.

Although the above discussion focused on the antimigraine agents ergotamine and propranolol, it will be appreciated that other antimigraine agents may also be utilized within the scope of the present invention. What is important is that the antimigraine agent be lipophilic, potent, and fast-acting so that the desired effects can be observed by the medical professional or by the patient himself, if the drug is to be self-administered, in sufficient time to remove the lollipop from the patient's mouth in time to prevent overdosing.

### D. Antiemetic Agents

Nausea and vomiting may occur independently of each other, but generally they are so closely related that they can be considered together. Nausea denotes the feeling of the imminent desire to vomit, while vomiting refers to the forceful oral expulsion of gastric contents. Nausea often precedes or accompanies vomiting and is usually associated with diminished functional activity of the stomach and alterations of the motility of the duodenum and small intestine. Increased perspiration, salivation, and the occasional association of hypotension and bradycardia often accompanies severe nausea.

The stomach plays a relatively passive role in the vomiting process, the major ejection force being provided by the abdominal musculature. Repeated emesis (vomiting) may have deleterious effects in a number of ways. The process of vomiting itself may lead to traumatic rupture or tearing of the region of the cardioesophageal junction, resulting in massive hematemesis (the vomiting of blood).

Prolonged vomiting may also lead to dehydration and the loss of gastric secretions, particularly hydrochloric acid, and metabolic alkalosis and the potentially dangerous loss of potassium. In states of central nervous system depression, such as coma, the gastric contents may actually be aspirated into the lungs, with a resulting aspiration pneumonitis.

The act of vomiting is under the control of two functionally distinct medullary (central nervous system) centers: the vomiting center and the chemoreceptor zone. The vomiting center controls and integrates the actual act of emesis, receiving stimuli from the intestinal tract, the labyrinthine apparatus in the ear, the chemoreceptor zone and other parts Of the body. The chemoreceptor trigger zone is also located in the medulla. Activation of this zone initiates impulses to the medullary vomiting center which then initiates the act of emesis. The chemoreceptor trigger zone can be activated by many stimuli, including drugs such as morphine, codeine, cardiac glycocides (digoxin) and ergot alkaloids (ergotamine) and a great many antineoplastic agents. Nausea and vomiting are common manifestations of organic and functional disorders.

Table II provides examples of many disorders which may be accompanied by nausea and vomiting.

**Table II**

| | |
|---|---|
| 1. | acute abdominal emergencies (i.e., acute appendicitis, cholecystitis) |
| 2. | chronic indigestion |
| 3. | acute infectious diseases accompanied with fever, especially in young children |
| 4. | disorders of the nervous system, especially the central nervous system |
| 5. | heart diseases such as acute myocardial infarction, especially of the posterior wall of the heart |
| 6. | metabolic and endocrine disorders, including diabetic acidosis |
| 7. | drugs and chemicals |
| 8. | emotional stress may lead to psychogenic vomiting |

As described above, nausea and subsequent vomiting is not only an undesirable sensation, it can be severely debilitating and even life threatening in some cases. There are currently only three modalities used to deliver the drugs to ameliorate nausea and vomiting. These are oral (tablets or liquids), intramuscular or intravenous injection, and rectal.

For rapid onset of antiemetic action, the intramuscular or intravenous route is preferred. But it is only rarely available outside of medical facilities, and even then, being an injection, it is the least appealing. The rectal route can be effective, but it is unpredictable at best, due to the variability and placement of the suppository and subsequent rapid liver metabolism. The oral route is the easiest, but as is usually the case, the antiemetic agent in tablet or solution is expectorated with the emesis before dissolution or absorption can take place.

A lollipop into which a suitable antiemetic agent has been dispersed when administered in a dose-to-effect manner provides a delivery method superior to current methods. Transmucosal administration provides for absorption of the antiemetic agent almost as fast as by the intravenous route. Having a handle, an antiemetic-containing lollipop may be quickly removed from the mouth in the case of unanticipated emesis. Further, transmucosal administration bypasses immediate hepatic metabolism which occurs with oral administration. More importantly, the antiemetic agent cannot be expectorated during transmucosal administration, which usually occurs after oral ingestion of an antiemetic agent. Finally, a drug-containing lollipop administered in a dose-to-effect manner is a highly acceptable mode of delivery.

Examples of compositions and methods of using lollipops containing antiemetic agents are given herein below.

### EXAMPLE 8

A drug-containing lollipop within the scope of the present invention to be used in the treatment of nausea and vomiting is made according to the procedure of Example 2, except that the ingredients are combined in the following amounts:

| Ingredient | % | grams |
|---|---|---|
| Natural mint | 1.0% | 0.2 |
| Ribotide | 1.5% | 0.3 |
| Aspartame | 2.0% | 0.4 |
| Meclizine | 2.5% | 0.5 |
| Wild cherry | 3.0% | 0.6 |
| Compritol 888 | 4.0% | 0.8 |
| Artificial vanilla | 5.0% | 1.0 |
| Artificial vanilla cream | 5.0% | 1.0 |
| Compressed sugar | 25.33% | 5.07 |
| Maltodextrin | 50.67% | 10.13 |

The foregoing procedure results in the preparation of 10 lollipops, each containing 50 milligrams of meclizine.

### EXAMPLE 9

In the procedure of this example, a patient who is presently experiencing nausea and vomiting is given a meclizine-containing lollipop in order to rapidly relieve the nausea and vomiting. In this example, meclizine in a lollipop dose of 50 milligrams is used. As the patient sucks on the lollipop, the distress associated with nausea and vomiting is eliminated.

Although the above discussion focused on the antiemetic agent meclizine, it will be appreciated that other antiemetic agents may also be utilized within the scope of the present invention. What is important is that the antiemetic agent be lipophilic, potent, and fast-acting so that the desired effects can be observed by the medical professional or by the patient himself, if the drug is to be self-administered, in sufficient time to remove the lollipop from the patient's mouth in time to prevent overdosing.

From the foregoing, it will be appreciated that the present invention allows great flexibility and permits physician control on a case-by-case basis with respect to the dose given to a particular patient, and the rate at which that dose is given.

The use of a drug-containing lollipop for administration of antimigraine, antiemetic or bronchodilator, agents is much faster acting than oral administration, and also avoids unacceptable loss of the drug on first pass through the liver before systemic distribution. Further, the use of the lollipop in accordance with the present invention provides for a relatively level drug plasma concentration, which is preferable when dealing with potent drugs.

Further, a physician can easily monitor a patient's condition to ensure the patient receives a dose adequate to evoke a desired physiological state. If necessary, the physician can instruct the patient to alter the aggressiveness with which he sucks the lollipop, or he can take the lollipop from the patient.

A patient can also self-administer suitable antimigraine, antiemetic, or bronchodilator medication using a lollipop in accordance with the present invention. Thus, a patient can place a drug-containing lollipop passively in his mouth for continuous low-level administration of a drug, or can take a lick of the lollipop from time to time as it may be needed to reduce his own subjective experience of pain or physical discomfort.

## Claims

1. A composition for use in treating in a dose-to-effect manner a patient experiencing pain associated with a migraine, said composition comprising:
an effective dose of a drug capable of being absorbed through mucosal tissues of the mouth, pharynx, and oesophagus, and capable of rapidly relieving the pain associated with a migraine;
a soluble matrix material, the drug being dispersed substantially uniformly within the matrix so that the drug is released in a dose-to-effect manner for absorption through mucosal tissues of the mouth, pharynx, and oesophagus as the matrix dissolves when placed in a patient's mouth; and
holder means secured to the drug-containing matrix, said holder means being configured so as to permit convenient insertion of the drug-containing matrix into the mouth of a patient and convenient removal thereof while accounting for the patient's susceptibility to the drug and the patient's individual subjective experience of the pain associated with the migraine.

2. A composition as defined in claim 1, wherein the drug is selected from the group comprising ergotamine, propranolol, methysergide and suloctidil.

3. A composition as defined in claim 1 or 2, wherein the drug and dosage are selected from the group comprising:
ergotamine and the dosage of ergotamine dispersed in the matrix is in the range from 2mg to 4mg of ergotamine equivalent;
propranolol and the dosage of propranolol dispersed in the matrix is in the range from 80mg to 160mg of propranolol equivalent;
methysergide and the dosage of methysergide dispersed in the matrix is in the range from 2mg to 4mg of methysergide equivalent;
suloctidil and the dosage of suloctidil dispersed in the matrix is in the range from 200mg to 300mg of suloctidil equivalent.

4. A composition for use in treating in a dose-to-effect manner a patient experiencing nausea and vomiting, said composition comprising:
an effective dose of an antiemetic drug capable of being absorbed through mucosal tissues of the mouth, pharynx, and oesophagus, and capable of rapidly relieving nausea and vomiting;
a soluble matrix material, the drug being dispersed substantially uniformly within the matrix so that the drug is released in a dose-to-effect manner for absorption through mucosal tissues of the mouth, pharynx, and oesophagus as the matrix dissolves when placed in a patient's mouth; and
holder means secured to the drug-containing matrix, said holder means being configured so as to permit convenient insertion of the drug-containing matrix, said holder means being configured so as to permit convenient insertion of the drug-containing matrix into the mouth of a patient and convenient removal thereof while accounting for the patient's susceptibility to the drug and the patient's individual subjective experience of nausea and vomiting.

5. A composition as claimed in claim 4 wherein the drug is selected from the group comprising meclizine, benzquinamide, metoclopramide and prochlorperazine.

6. A composition as defined in claim 5 wherein the drug and dosage are selected from the group comprising:
meclizine and the dosage of meclizine dispersed in the matrix is in the range from 25mg to 100mg of meclizine equivalent;
benzquinamide and the dosage of benzquinamide dispersed in the matrix is in the range from 25mg to 100mg of benzquinamide equivalent;
metoclopramide and the dosage of metoclopramide dispersed in the matrix is in the range from 5mg to 20mg of metoclopramide equivalent;
prochlorperazine and the dosage of prochlorperazine dispersed in the matrix is in the range from 5mg to 25mg of prochlorperazine equivalent; and

7. A composition for use in treating in a dose-to-effect manner a patient experiencing a respiratory distress, said composition comprising:
an effective dose of a drug capable of being absorbed through mucosal tissues of the mouth, pharynx, and oesophagus, and capable of rapidly relieving the respiratory distress;
a soluble matrix material, the drug being dispersed substantially uniformly within the matrix so that the drug is released in a dose-to-effect manner for absorption through mucosal tissues of the mouth, pharynx, and oesophagus as the matrix dissolves when placed in a patient's mouth; and
holder means secured to the drug-containing matrix, said holder means being configured so as to permit convenient insertion of the drug-containing matrix into the mouth of a patient and convenient removal thereof while accounting for the patient's susceptibility to the drug and the patient's individual subjective experience of the respiratory distress.

8. A composition as claimed in claim 10 wherein the drug is selected from the group comprising albuterol, aminophylline, beclomethasone, dyphylline, epinephrine, flunisolide, isoetharine, isoproterenol hydrochloride, metaproterenol, oxytriphylline, terbutaline and theophylline.

9. A composition as defined in claim 11 wherein the drug is oxytriphylline and the dosage of oxytriphylline dispersed in the matrix is in the range from 50mg to 400mg of oxytriphylline equivalent.

10. A composition as defined in claim 12 wherein the drug is theophylline and the dosage of theophylline dispersed in the matrix is in the range from 50mg to 400mg of theophylline equivalent.

11. A composition as defined in any preceding claim, wherein the drug containing lollipop includes an inner matrix containing the drug in a suitable concentration for maintaining the desired systemic relief and an outer matrix covering the inner matrix, said outer matrix containing the drug in a suitable concentration more rapidly producing the desired systemic relief.

12. A composition as defined in any preceding claim, wherein the holder means is a stick with an enlarged end to prevent it from getting caught in a patient's mouth.

13. A composition as defined in any preceding claim wherein the dosage of drug dispersed in the soluble matrix material is in the range from 1 to 50 times greater than the dosage which would be given by intravenous injection.

14. A composition as claimed in any preceding claim adapted to permit a patient to exert control over the dosage and rate of the drug received.

## Patentansprüche

1. Zusammensetzung zur Verwendung bei der Behandlung in einer dosisabhängigen Art und Weise eines Patienten, der an mit Migräne einhergehendem Schmerz leidet, welche umfaßt:
eine wirksame Dosis eines Arzneimittels, das durch die Schleimhaut des Mundes, des Rachens und der Speiseröhre aufgenommen werden kann und den mit Migräne einhergehenden Schmerz schnell lindern kann,
ein lösliches- Trägermatrix-Material, wobei das Arzneimittel in der Trägermatrix im wesentlichen einheitlich dispergiert ist, so daß das Arzneimittel in einer dosisabhängigen Art und Weise zur Absorption durch die Schleimhäute des Mundes, des Rachens und der Speiseröhre beim Auflösen der Matrix bei Überführung in den Mund des Patienten das Arzneimittel freisetzt, und
Haltemittel, die an der das Arzneimittel enthaltenden Matrix angebracht sind, wobei die Haltemittel so ausgestaltet sind, daß sie eine zweckmäßige Einführung der das Arzneimittel enthaltenden Matrix in den Mund eines Patienten und eine zweckmäßige Entfernung davon ermöglichen, wobei die Empfänglichkeit des Patienten gegenüber dem Arzneimittel und die individuelle subjektive Erfahrung des mit Migräne einhergehenden Schmerzes berücksichtigt werden kann.

2. Zusammensetzung nach Anspruch 1,
bei der das Arzneimittel ausgewählt ist aus der Gruppe enthaltend Ergotamin, Propanolol, Methysergid und Suloctidil.

3. Zusammensetzung nach Anspruch 1 oder 2,
bei der das Arzneimittel und die Dosis ausgewählt werden aus der Gruppe umfassend:
Ergotamin, wobei die Dosis des in der Matrix dispergierten Ergotamins im Bereich von 2 mg bis 4 mg Ergotaminäquivalent liegt,
Propanolol, wobei die Dosis des in der Matrix dispergierten Propanolols im Bereich von 80 mg bis 160 mg Propanololäquivalent liegt,
Methysergid, wobei die Dosis des in der Matrix dispergierten Methysergids im Bereich von 2 mg bis 4 mg Methysergidäquivalent liegt,
Suloctidil, wobei die Dosis des in der Matrix dispergierten Suloctidils im Bereich von 200 mg bis 300 mg Suloctidiläquivalent liegt.

4. Zusammensetzung zur Verwendung bei Behandlung in einer dosisabhängigen Art und Weise eines an Übelkeit und Erbrechen leideten Patienten, welche umfaßt:
eine wirksame Dosis eines Antibrechmittels, welches durch die Schleimhäute des Mundes, des Rachens und der Speiseröhre aufgenommen und Übelkeit und Erbrechen schnell lindern kann,
ein lösliches Trägermatrixmaterial, wobei das Arzneimittel in der Trägermatrix im wesentlichen einheitlich dispergiert ist, so daß das Arzneimittel in einer dosisabhängigen Art und Weise zur Absorption durch die Schleimhäute des Mundes, des Rachens und der Speiseröhre beim Auflösen der Matrix bei Überführung in den Mund eines Patienten freigesetzt wird, und
Haltemittel, die an die das Arzneimittel enthaltenden Trägermatrix angebracht sind, wobei die Haltemittel so ausgestaltet sind, daß sie eine zweckmäßige Einführung der das Arzneimittel enthaltenden Matrix in den Mund eines Patienten und eine zweckmäßige Entfernung davon ermöglichen, wobei die Empfänglichkeit des Patienten gegenüber dem Arzneimittel und die individuelle subjektive Erfahrung der Übelkeit und des Erbrechens des Patienten berücksichtigt werden kann.

5. Zusammensetzung nach Anspruch 4,
bei der das Arzneimittel ausgewählt ist aus der Gruppe umfassend Meclizin, Benzquinamid, Metoclopramid und Prochlorperazin.

6. Zusammensetzung nach Anspruch 5,
bei der das Arnzeimittel und die Dosis aus der Gruppe ausgewählt werden umfassend:
Meclizin, wobei die Dosis des in der Matrix dispergierten Meclizin im Bereich von 25 mg bis 100 mg Meclizinäquivalent liegt,
Benzquinamid, wobei die Dosis des in der Matrix dispergierten Benzquinamid im Bereich von 25 mg bis 100 mg Benzquinamidäquivalent liegt,
Metoclopramid, wobei die Dosis des in der Matrix dispergierten Metoclopramids im Bereich von 5 mg bis 20 mg Metoclopramidäquivalent liegt,
Prochlorperazin, wobei die Dosis des in der Matrix dispergierten Prochlorperazins im Bereich von 5 mg bis 25 mg Prochlorperazinäquivalent liegt.

7. Zusammensetzung zur Verwendung bei der Behandlung in einer dosisabhängigen Art und Weise eines an Atemnot leidenden Patienten, welche umfaßt:
eine wirksame Dosis eines Arzneimittels, das durch die Schleimhaut des Mundes, des Rachens und der Speiseröhre aufgenommen werden kann und den mit einer Atemnot einhergehenden Schmerz schnell lindern kann,
ein lösliches Trägermatrix-Material, wobei das Arzneimittel in der Trägermatrix im wesentlichen einheitlich dispergiert ist, so daß das Arzneimittel in einer dosisabhängigen Art und Weise zur Absorption durch die Schleimhäute des Mundes, des Rachens und der Speiseröhre beim Auflösen der Matrix bei Überführung in den Mund des Patienten das Arzneimittel freisetzt, und
Haltemittel, die an der das Arzneimittel enthaltenden Matrix angebracht sind, wobei die Haltemittel so ausgestaltet sind, daß sie eine zweckmäßige Einführung der das Arzneimittel enthaltenden Matrix in den Mund eines Patienten und eine zweckmäßige Entfernung davon ermöglichen, wobei die Empfänglichkeit des Patienten auf das Arzneimittel und die individuelle subjektive Erfahrung des mit der Atemnot einhergehenden Schmerzes berücksichtigt werden kann.

8. Zusammensetzung nach Anspruch 7,
wobei das Arzneimittel ausgewählt ist aus der Gruppe umfassend Albuterol, Aminophyllin, Beclomethason, Dyphyllin, Epinephrin, Flunisolid, Isoetharin, Isoproterenolhydrochlorid, Metaproterenol, Oxytriphyllin, Terbutalin und Theophyllin.

9. Zusammensetzung nach Anspruch 8,
wobei das Arzneimittel Oxytriphyllin und die Dosis des in der Matrix dispergierten Oxytriphyllin im Bereich von 50 mg bis 400 mg Oxytriphyllinäquivalente liegt.

10. Zusammensetzung nach Anspruch 9,
wobei das Arzneimittel Theophyllin und die Dosis des in der Matrix dispergierten Theophyllins im Bereich von 50 mg bis 400 mg Theophyllinäquivalent liegt.

11. Zusammensetzung nach einem der vorhergehenden Ansprüche,
wobei der das Arzneimittel enthaltende Lutscher eine innere, das Arzneimittel in einer geeigneten Konzentration enthaltende Matrix zum Aufrechterhalten der gewünschten systemischen Linderung und eine äußere Matrix enthält, die die innere Matrix umgibt, wobei die äußere Matrix das Arzneimittel in einer geeigneten Konzentration enthält, die die gewünschte systemische Linderung schneller hervorruft.

12. Zusammensetzung nach einem der vorhergehenden Ansprüche,
bei der das Haltemittel ein Stiel mit einem vergrößerten Ende ist, damit er nicht in dem Mund des Patienten hängen bleibt.

13. Zusammensetzung nach einem der vorhergehenden Ansprüche,
bei der die Dosis des in dem löslichen Trägermatrix-Material dispergierten Arzneimittels im Bereich von 1 bis 50 mal über der Dosis liegt, die über intravenöse Injektion verabreicht werden würde.

14. Zusammensetzung nach einem der vorhergehenden Ansprüche,
die dazu angepaßt ist, einem Patienten die Steuerung über die Dosis und die Menge des erhaltenen Arzneimittels zu ermöglichen.

## Revendications

1. Composition destinée à être utilisée dans le traitement d'une manière dose-effet d'un patient qui est en train de ressentir une douleur associée à une migraine, ladite composition comprenant :
une dose efficace d'un médicament capable d'être absorbé à travers les tissus muqueux de la bouche, du pharynx et de l'oesophage, et capable de rapidement soulager la douleur associée à une migraine;
une matière de matrice soluble, le médicament étant dispersé de manière sensiblement uniforme à l'intérieur de la matrice de façon à ce que le médicament soit libéré d'une manière dose-effet pour une absorption à travers les tissus muqueux de la bouche, du pharynx et de l'oesophage à mesure que la matrice se dissout quand elle est placée dans la bouche d'un patient ; et
un moyen de tenue fixé à la matrice contenant le médicament, ledit moyen de tenue étant configuré de façon à permettre l'introduction pratique de la matrice contenant le médicament dans la bouche d'un patient et le retrait pratique de celle-ci tout en prenant en compte la sensibilité du patient au médicament et la perception subjective individuelle du patient de la douleur associée à la migraine.

2. Composition selon la revendication 1, dans laquelle le médicament est choisi dans le groupe constitué de l'ergotamine, du propranolol, du méthysergide et du suloctidil.

3. Composition selon la revendication 1 ou 2, dans laquelle le médicament et la dose sont choisis dans le groupe comprenant :
l'ergotamine, et la dose d'ergotamine dispersée dans la matrice étant comprise entre 2 mg et 4 mg d'équivalent d'ergotamine;
le propranolol, et la dose de propranolol dispersée dans la matrice étant comprise entre 80 mg et 160 mg d'équlvalent de propranolol;
le méthysergide, et la dose de méthysergide dispersée dans la matrice étant comprise entre 2 mg et 4 mg d'équivalent de méthysergide;
le suloctidil, et la dose de suloctidil dispersée dans la matrice étant comprise entre 200 mg et 300 mg d'équivalent de suloctidil.

4. Composition destinée à être utilisée dans le traitement d'une manière dose-effet d'un patient qui est en train de ressentir des nausées et de vomir, ladite composition comprenant :
une dose efficace d'un médicament antiémétique capable d'être absorbé à travers les tissus muqueux de la bouche, du pharynx et de l'oesophage, et capable de rapidement calmer les nausées et les vomissements;
une matière de matrice soluble, le médicament étant dispersé de manière sensiblement uniforme à l'intérieur de la matrice de façon à ce que le médicament soit libéré d'une manière dose-effet pour une absorption à travers les tissus muqueux de la bouche, du pharynx et de l'oesophage à mesure que la matrice se dissout quand elle est placée dans la bouche d'un patient ; et
un moyen de tenue fixé à la matrice contenant le médicament, ledit moyen de tenue étant configuré de façon à permettre l'introduction pratique de la matrice contenant le médicament dans la bouche d'un patient et le retrait pratique de celle-ci tout en prenant en compte la sensibilité du patient au médicament et la perception subjective individuelle du patient des nausées et des vomissements.

5. Composition selon la revendication 4 dans laquelle le médicament est choisi dans le groupe constitué de la méclizine, du benzquinamide, du métoclopramide et de la prochlorpérazine.

6. Composition selon la revendication 5 dans laquelle le médicament et la dose sont choisis dans le groupe comprenant :
la méclizine, et la dose de méclizine dispersée dans la matrice étant comprise entre 25 mg et 100 mg d'équivalent de méclizine;
le benzquinamide, et la dose de benzquinamide dispersée dans la matrice étant comprise entre 25 mg et 100 mg d'équivalent de benzquinamide;
le métoclopramide, et la dose de métoclopramide dispersée dans la matrice étant comprise entre 5 mg et 20 mg d'équivalent de métoclopramide;
la prochlorpérazine, et la dose de prochlorpérazine dispersée dans la matrice étant comprise entre 5 mg et 25 mg d'équivalent de prochlorpérazine.

7. Composition destinée à être utilisée dans le traitement d'une manière dose-effet d'un patient qui est en train d'éprouver une détresse respiratoire, ladite composition comprenant :
une dose efficace d'un médicament capable d'être absorbé à travers les tissus muqueux de la bouche, du pharynx et de l'oesophage, et capable de rapidement soulager la détresse respiratoire;
une matière de matrice soluble, le médicament étant dispersé de manière sensiblement uniforme à l'intérieur de la matrice de façon à ce que le médicament soit libéré d'une manière dose-effet pour une absorption à travers les tissus muqueux de la bouche, du pharynx et de l'oesophage à mesure que la matrice se dissout quand elle est placée dans la bouche d'un patient ; et
un moyen de tenue fixé à la matrice contenant le médicament, ledit moyen de tenue étant configuré de façon à permettre l'introduction pratique de la matrice contenant le médicament dans la bouche d'un patient et le retrait pratique de celle-ci tout en prenant en compte la sensibilité du patient au médicament et la perception subjective individuelle du patient de la détresse respiratoire.

8. Composition selon la revendication 7 dans laquelle le médicament est choisi dans le groupe constitué de l'albutérol, l'aminophylline, la béclométhasone, la dyphylline, l'épinéphrine, le flunisolide, l'isoétharine, le chlorhydrate d'isoprotérénol, le métaprotérénol, l'oxytriphylline, la terbutaline, et la théophylline.

9. Composition selon la revendication 8 dans laquelle le médicament est l'oxytriphylline et la dose d'oxytriphylline dispersée dans la matrice est comprise entre 50 mg et 400 mg d'équivalent d'oxytriphylline.

10. Composition selon la revendication 8 dans laquelle le médicament est la théophylline et la dose de théophylline dispersée dans la matrice est comprise entre 50 mg et 400 mg d'équivalent d'oxytriphylline.

11. Composition selon l'une quelconque des revendications précédentes, dans laquelle la sucette contenant le médicament comprend une matrice interne contenant le médicament à une concentration appropriée pour maintenir le soulagement systémique désiré et une matrice externe recouvrant la matrice interne, ladite matrice externe contenant le médicament à une concentration appropriée produisant plus rapidement le soulagement systémique désiré.

12. Composition selon l'une quelconque des revendications précédentes, dans laquelle le moyen de tenue est un bâtonnet ayant une extrémité élargie pour l'empêcher d'être happé dans la bouche d'un patient.

13. Composition selon l'une quelconque des revendications précédentes dans laquelle la dose de médicament dispersée dans la matière de matrice soluble est de 1 à 50 fois plus élevée que la dose qui aurait été donnée par injection intraveineuse.

14. Composition selon l'une quelconque des revendications précédentes adaptée de façon à permettre à un patient d'exercer un contrôle sur la dose et la fréquence de prise du médicament reçu.
